# EUROPEAN PATENT SPECIFICATION

(11) **EP 0 260 707 B1**
(45) Date of publication and mention of the grant of the patent: **27.01.1993**
(21) Application number: 87113681.8
(22) Date of filing: 18.09.1987
(51) Int. Cl.: C12Q 1/56

(54) **Method for assaying plasma protein and measuring kit for the same**
Verfahren zur Bestimmung von Plasma-Protein und Testsatz dafür
Méthode pour l'essai de protéine du plasma et trousse à réactifs à cet effet

(30) Priority: 19.09.1986 JP 222294/86; 18.02.1987 JP 36739/87
(43) Date of publication of application: 23.03.1988
(73) Proprietor: NIPPON ZOKI PHARMACEUTICAL CO. LTD., Osaka-shi Osaka-fu (JP)
(72) Inventor: Matsumoto, Kenji, c/o Inst. of Bio-Active Science, Kinashi, Hyogo (JP)
(74) Representative: Hansen, Bernd, Dr. Dipl.-Chem.

(56) References cited:
- DE-A- 3 536 903
- FR-A- 2 378 004
- CHEMICAL ABSTRACTS, vol. 100, no. 19, 07 May 1984, Columbus, OH (US); N.SALA et al., p. 216, no. 152824t
- BIOCHEMISTRY, vol. 23, 1984, American Chemical Society, Easton, PA (US); R.KIKUMOTO et al., pp. 85-90
- CHEMICAL ABSTRACTS, vol. 93, no. 11, 15 September 1980, Columbus, OH (US); R.KIKUMOTO et al., p. 24, no. 106806c; S.OKAMOTO et al., p. 25, no. 106807d
- CHEMICAL ABSTRACTS, vol. 100, no. 11, 12 March 1984, Columbus, OH (US); P.C.COMP et al., p. 208, no. 81654z

## Description

### BACKGROUND OF THE INVENTION

This invention relates to a method for assaying protein C ( hereinafter abbreviated as PC ) and to a kit for measuring PC activity. More particularly, it relates to an enzymatic method for directly measuring the activity of PC without isolating it from the plasma, and to a measuring kit for the same.

PC is a vitamin-K-dependent plasma protein, which can be converted to activated protein C ( hereinafter abbreviated as PCa ) by the action of thrombin-thrombomodulin complex in the presence of Ca²⁺ ions. PCa is receiving attention as a plasma protein that is capable of selectively degrading blood coagulation factors V and VIII, thus exhibiting a powerful anti-coagulant action, and of liberating vascular plasminogen activator, thereby accelerating fibrinolysis.

It is known that PC level in the blood decreases as a result of disseminated intravascular coagulation syndrome ( DIC ) and heptic diseases, such as hepatocirrhosis and chronic hepatitis. Recently a new PC deficiency disease accompanied by multiple thrombosis was reported. In addition, the effect of PC content upon the efficacy of blood coagulation factor ( e.g., factor IX ) preparations has been a subject of major concern.

Under the circumstances, there has been a demand for a simple method for correctly assaying PC in the plasma to diagnose the aforementioned PC deficiency disease, DIC and hepatic diseases, and to check the efficacy of various blood coagulation factor preparations.

Several methods are now used for quantitative analysis of PC, including the enzymatic method which employs a synthetic peptide substrate specific to PCa. But direct measurement of the activity of PC in the plasma by this type of method has been difficult, because the blood contains PC inhibitors and other interfering substances which, during activity measurement, act upon the synthetic peptide substrate without being inhibited by antithrombin III.

It is therefore necessary to previously isolate PC from the plasma by means of an antibody column or an adsorbent, as disclosed e.g. in Chem. Abstr., Vol. 100/19, page 216, n° 152824t and BLOOD 1984, 63(3), pages 671-675 : "A functional assay of protein C in human plasma". But such a pretreatment, which requires a considerable amount of sample blood and needs much time and labor, is unsuitable for rapid treatment of a large number of samples. In addition, the use of an adsorbent suffers from the low recovery rate of PC, while the use of an antibody column is very costly, although the PC recovery rate is satisfactory.

A biological method utilizing the anticoagulating action of PC is also known, in which PC, after being activated with snake venom, is added to a blood coagulation system and its action to prolong the coagulation time is measured. But the sensitivity of this method is not so high.

Immunological methods are also known, in which the amount of PC antigen is measured through an antigen-antibody reaction using a polyclonal or monoclonal antibody ( the radioimmunoassay, enzyme immunoassay and Laurell's method ). The techniques of this type are capable of correctly measuring the amount of PC antigen, but the disadvantages are that the antibody used is very costly, long time is needed for measurement, and it is impossible to judge whether the PC tested has normal activity or not.

As a result of intensive studies to establish a new analytical method for PC free of the above-mentioned problems, we have succeeded in accomplishing this invention.

### SUMMARY OF THE INVENTION

An object of this invention is to provide a new and useful method of assaying PC.

A further object of this invention is to provide an enzymatic method for measuring PC activity which allows treatment of a large number of samples in a short time by simple operations without pretreatment to isolate PC from the plasma.

Another object of this invention is to provide a measuring kit to be used in the above-mentioned method.

The PC assaying method of this invention is an enzymatic method of measuring PC activity by the use of a synthetic peptide substrate, wherein interfering substances acting upon said peptide substrate are specifically inhibited.

The PC activity measuring kit of this invention comprises (A) a PC activator, (B) antithrombin III, (C) a thrombin inhibitor having a molecular weight of no more than 2000, and (D) a synthetic peptide substrate.

### BREIF DESCRIPTION OF THE DRAWINGS

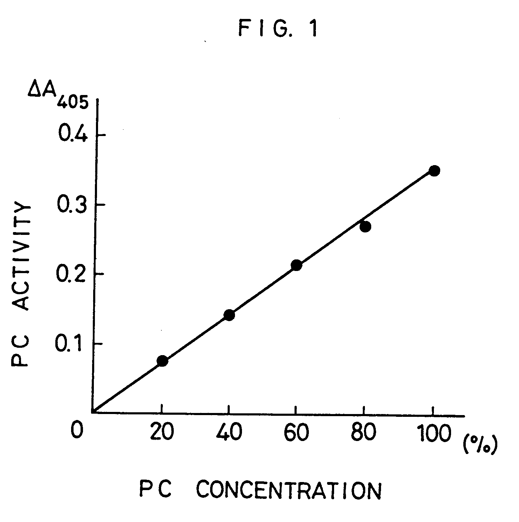

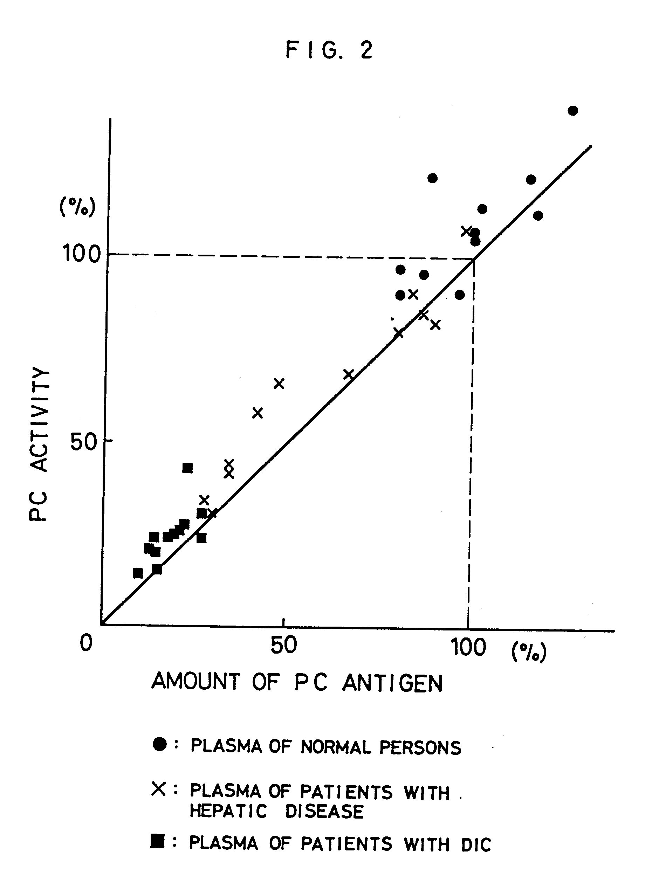

Figure 1 shows a calibration curve prepared according to the data obtained in the Example of this invention, and Figure 2 is a graph illustrating a relationship between the method of this invention and the enzyme immunoassay ( EIA ).

### DETAILED DESCRIPTION OF THE INVENTION

In the PC assaying method of this invention, interfering substances ( other than PCa ) contained in the plasma and acting upon the synthetic peptide substrate are inhibited, thereby allowing direct measurement of PC activity without isolating it from the plasma.

The method of this invention comprises the steps of :
diluting a plasma sample with a buffer solution by a factor of 10 or more,
adding a PC activator to the diluted plasma sample to form activated PC (hereinafter referred to as PCa),
adding an antithrombin III and a thrombin inhibitor having a molecular weight of no more than 2000,
adding a synthetic peptide substrate, and colorimetrically or fluorometrically measuring the amount of decomposition product formed by the action of PCa.

The method of this invention is described below in more detail.

### 1) Plasma dilution rate

Since the plasma contains the endogenous PC inhibitors which inhibit the action of PCa, PCa activity cannot be correctly determined without taking suitable measures to eliminate its effect. In the method of this invention, the effect of PC inhibitor can be avoided by diluting the plasma sample by a factor of 10 or more; preferably by a factor of 10 to 30.

The plasma used in this invention may any plasma sample prepared by commonly employed methods, for example, citrate-containing plasma prepared by taking a blood sample in the presence of sodium citrate, followed by centrifugal separation.

### 2) Buffer solution

A buffer solution adjusted to a pH level near the conditions in living bodies by using a buffering agent, such as Tris-HCl, should preferably be used to dilute the plasma. Sodium chloride and other salts may also be added to the solution, as required, to bring it more close to the conditions in living bodies. In addition, protease inhibitors, such as soybean trypsin inhibitor ( SBTI ) and aprotinin, may also be added in amounts that will not affect PCa activity in order to suppress the actions of various proteases in the plasma.

Furthermore, a stabilizer, such as bovine serum albumin ( BSA ), may also be added to prevent precipitation of proteins under weakly acidic conditions at the termination of the reaction.

### 3) PC activator

Thrombin-thrombomodulin complex, snake venom and others may be mentioned as the PC activator, but use of thrombin-thrombomodulin complex is preferable in terms of physiologically natural conditions. It may be added in the form of complex, or thrombin and thrombomodulin may be added separately.

Since activation of PC requires the presence of Ca²⁺ ions, it is preferable to add these ions to the buffer solution or to the solution of PC activator in an amount that will give optimum concentration in the PC activation system.

### 4) Inhibitor against interfering substances

Thrombin added to activate PC also acts upon the synthetic peptide substrate, and hence interferes with the measurement of PC activity. It is therefore necessary to add an inhibitor against thrombin after PC has been activated. This inhibitor must preferably be such that it inhibits the action of thrombin and shows no effect upon PCa. Antithrombin III may be used for this purpose; in actual practice, it is preferable to use heparin in combination because it markedly accelerates the action of antithrombin III.

However, addition of antithrombin III alone cannot inhibit the activity of the other interfering substances contained in the plasma. In fact, measurement of PC activity by the use of a synthetic substrate in combination with antithrombin III/heparin alone gave an unusually high apparent PC activity - multi-hundred times as high as the estimated level obtained by the immunological method. Thus, previous isolation of PC by the use of an antibody column or an adsorbent, or other intricate pretreatment, is indispensable when measuring PC activity by addition of antithrombin III alone.

Assiduous studies in pursuit of an effective method for inhibiting interfering substances acting upon the synthetic substrate have led us to find that this object an be achieved by using a low-molecular thrombin inhibitor in combination with high-molecular substances like antithrombin III, and to succeed in correctly measuring PC activity without the need for isolation and purification of PC.

The low-molecular thrombin inhibitors that can be used in the method of this invention are preferably those which show no effect upon the activity of PCa. These include thrombin inhibitors with a molecular weight not higher than 2,000, more preferably, not higher than 1,000. Illustrative examples are N-arylsulfonyl-L-arginine derivatives, such as (2R,4R)-1-[N²-(3-methyl-1,2,3,4-tetrahydro-8-quinolinesulfonyl)-L-arginyl]-4-methyl-2-piperidinecarboxylic acid ( Compound 1 ) and dansylarginine N-(3-ethyl-1,5-pentanediyl)amide ( Compound 2 ) [ J. Med. Chem., 23, 827-836 and 1293-1299 ( 1980 ), and others ]; and N-arylsulfonylglycyl-amidinophenylalanine derivatives, such as N^{α}-aryl-(2-naphthylsulfonylglycyl)-4-amidinophenylalanine-piperidide ( Compound 3 ) [ Thromb. Res., 36, No.5, 457-465 ( 1984 )].

### 5) Synthetic peptide substrate

Synthetic peptide substrates commonly used in conventional methods for measuring enzymatic activity can be employed in the method of this invention. Illustrative examples are color-developing synthetic substrates, such as Pyr-Pro-Arg-pNA ( S-2366: pyroglutamyl-prolyl-arginyl-p-nitroanilide ), D-Val-Leu-Arg-pNA ( S-2266: D-valyl-leucyl-arginyl-p-nitroanilide ) and D-Phe-Pip-Arg-pNA ( S-2238: D-phenylalanyl-piperidino-arginyl-p-nitroanilide ); and fluorescent synthetic peptide substrates, such as Boc-Leu-Ser-Thr-Arg-MCA ( 3112-V: t-butoxycarbonylleucyl-seryl-threonyl-arginyl-4-methylcoumalinamide ).

The enzymatic reaction between PCa and a synthetic peptide substrate can be terminated by making the reaction system weakly acidic. Hence a weak acid, such as citric acid and acetic acid, is preferably used as the terminator.

This invention also relates to a PC activity measuring kit. As may be seen from the foregoing, this kit comprises (A) a PC activator, (B) antithrombin III, (C) a thrombin inhibitor having a molecular weight of no more than 2000, and (D) a synthetic peptide substrate.

Use of this PC activity measuring kit may be summarized as follows:
(a) A PC activator is added to diluted plasma to form PCa.
(b) The inhibitor against the action of interfering substances other than PCa ( antithrombin III and a low-molecular thrombin inhibitor ) is further added.
(c) A synthetic peptide substrate is then added to measure PC activity enzymatically. After termination of the reaction, the decomposition product formed by the action of PCa is colorimetrically or fluorometrically measured, thereby determing the amount of PC.

The following Example will further illustrate the invention.

### EXAMPLE

### - Kit components ( amounts in each vial ) -

(A) Thrombin-thrombomodulin complex ( freeze-dried product ) 10U/ml Thrombin/0.85nmol/ml thrombomodulin
   Dissolve in 4 ml distilled water for use.
(B) Antithrombin III/heparin complex ( freeze-dried product ) 25U/ml Antithrombin III/100U/ml heparin
   Dissolve in 4 ml of the following solution for use.
(C) Aqueous Solution of Compound 1 5 ml ( 100 µg/ml )
(D) S-2366 ( freeze-dried product )
   Dissolve in 4 ml distilled water for use ( 3mM aqueous solution ).

### -Operating procedure-

(a) Plasma being tested was diluted 1:20 with the buffer solution ( 50mM Tris-HCl(pH8.0)/0.1M NaCl/1mM CaCl₂/0.1% BSA/0.1mg/ml SBTI), the diluted solution was incubated at 56°C for five minutes, the fibrin which separated out was removed by centrifugation, and 200 µl of the supernatant was collected.
(b) The solution of thrombin/thrombomodulin complex ( 100 µl ) was added, and the mixture was incubated at 37°C for thirty minutes.
(c) The solution of antithrombin III/heparin/Compound 1 ( 100 µl ) was added, and the mixture was incubated at 37°C for five minutes.
(d) The solution of S-2366 ( 100 µl ) was added, and the mixture was incubated at 37°C for 30 minutes.
(e) A 2% aqueous solution of citric acid was added, and the absorbance at 405 nm was measured.

Thus, forty test samples can be assayed by one set of the measuring kit shown above. It is needless to say that various types of kits may also be prepared to meet particular needs by changing the amount of each component in each vial, by adjusting the plasma dilution rate and by appropriately altering the scale of the whole system.

### -Calibration curve-

A calibration curve prepared from pooled plasma of normal human adults is shown in Figure 1.

As is apparent from the figure, a linear relationship holds over the PC concentration range from 0 to 100%, indicating the high accuracy of PC activity determination by the use of the measuring kit of this invention.

Described below is the result of our studies on various measuring conditions with the kit of this invention. Fundamental operations are the same as adopted in the above Example.

### (1) Plasma dilution rate

A study on the relationship between the effect of PC inhibitor and plasma dilution rate has revealed that a linear relationship between plasma dilution rate and PC activity measured can be achieved if sample plasma is diluted by a factor of about 10 or higher. As the plasma dilution rate falls below this level, the action of PC inhibitor becomes more marked, with the curve deviating from linearity.

It is therefore preferable that the plasma sample be diluted by a factor of 10 or higher.

### (2) Ca²⁺ ion concentration

A test was made to determine the optimum Ca²⁺ ion concentration for PC activation. It was demonstrated that the concentration of Ca²⁺ in the buffer solution for diluting plasma should preferably be in the range of 0.1 to 4mM, most preferably in the range of 0.5 to 2.5mM.

### (3) Amount of thrombin added

In the reaction system of the above Example, PC could be activated almost quantitatively by the use of 1 U or more of thrombin. The amount of thrombin to be added was set to 1 U in the kit of the above Example in order to minimize the amount of antithrombin/heparin to be used after activation of PC.

### (4) Amount of thrombomodulin added

A test using different amounts of thrombomodulin revealed that PC could be sufficiently activated in the reaction system of the above Example if 0.04 nmol or more of thrombomodulin is added. The preferred amount is 0.06 to 0.16 nmol.

### (5) Amount of Compound 1 added

A study using Compound 1 with different concentrations showed that the effect of interfering substances that act upon the synthetic substrate S-2366 ( other than PCa ) could be eliminated almost completely, if the concentration of Compound 1 in the solution is about 75 µg/ml or more.

A separate experiment using isolated and purified PC showed that use of Compound 1 with a concentration of 150 µg/ml or more can affect PC activity. Hence, Compound 1 should preferably be used at a concentration lower than 150 µg/ml.

It was experimentally verified that Compound 2 may be used in much the same way as with Compound 1. Compound 3, on the other hand, could inhibit the activity of interfering substances almost completely at its solution concentration of about 25 µg/ml.

PC activity and amounts of PC antigen in the plasma of normal persons, patients suffering from a hepatic disease and patients suffering from DIC were measured by the method of this invention and by the immunological method [ EIA: J. C. Giddings et al., Brit. J. Haematol., 52, 495-502 ( 1982 )]. The results are summarized in Figure 2.

The PC activity data of the method of this invention were obtained from the calibration curve of Figure 1 and expressed in percentage.

Figure 2 clearly shows a good correlation between the PC activity measured by the method of this invention and the amount of PC antigen measured by the immunological method. This correlation holds not only for normal persons, but also for patients suffering hepatitis and DIC in which the content of plasma PC is generally lower.

The method of this invention dispenses with the pretreatment for isolation of PC, which is indispensable in the conventional enzymatic methods and needs much time and labor, thus allowing PC activity measurement by use of untreated plasma.

The volume of sample plasma required for each test is as small as 10 µl or less, and the amount of PC contained in it can be quantitatively determined in a short time by simple operations. The PC activity measured by the method of this invention correlates well with the amount of PC antigen measured by the immunological method, indicating the high accuracy of the method of this invention.

As is apparent from the foregoing, the method of this invention is very simple, accurate and rapid compared with the conventinal methods, and is particularly suitable for treatment of a vast number of samples. It is therefore very useful for diagnosis of PC deficiency diseases, DIC and hepatitis, and for testing the efficacy of various coagulation factor ( e.g., factor IX ) preparations.

## Claims

1. An enzymatic method for assaying protein C (hereinafter referred to as PC) which comprises the steps of:
diluting a plasma sample with a buffer solution by a factor of 10 or more,
adding a PC activator to the diluted plasma sample to form activated PC (hereinafter referred to as PCa),
adding an antithrombin III and a thrombin inhibitor having a molecular weight of no more than 2000,
adding a synthetic peptide substrate, and colorimetrically or fluorometrically measuring the amount of decomposition product formed by the action of PCa.

2. The method for assaying PC as defined in claim 1, wherein said PC activator is a thrombin-thrombomodulin complex.

3. The method for assaying PC as defined in claim 1, wherein heparin is used in combination with antithrombin III.

4. The method for assaying PC as defined in claim 1, wherein said thrombin inhibitor is an N²-arylsulfonyl-L-argininamide.

5. The method for assaying PC as defined in claim 1 wherein said thrombin inhibitor is an N-arylsulfonylglycyl-amidinophenylalanine.

6. A kit for measuring PC activity comprising (A) a PC activator, (B) antithrombin III, (C) a thrombin inhibitor having a molecular weight of no more than 2000, and (D) a synthetic peptide substrate.

7. The kit for measuring PC activity as defined in claim 6, wherein said thrombin inhibitor is an N²-arylsulfonyl-L-argininamide.

8. The kit for measuring PC activity as defined in claim 6, wherein said thrombin inhibitor is an N-arylsulfonylglycyl-amidinophenylalanine.

9. The kit for measuring PC activity as defined in claim 6, wherein the kit further comprises a buffer solution for diluting plasma and/or a reaction terminator.

10. The kit for measuring PC activity as defined in claim 9, wherein said PC activator is thrombin/thrombomodulin complex.

11. The kit for measuring PC activity as defined in claim 10, wherein heparin is used in combination with antithrombin III.

## Patentansprüche

1. Enzymatisches Verfahren zur Bestimmung von Protein C (nachfolgend als PC bezeichnet), umfassend folgende Stufen:
Verdünnen einer Plasmaprobe mit einer Pufferlösung um den Faktor 10 oder mehr,
Zugabe eines PC-Aktivators zu der verdünnten Plasmaprobe unter Ausbildung von aktiviertem PC (nachfolgend als PCa bezeichnet),
Zugabe von Antithrombin III - und eines Thrombininhibitors mit einem Molekulargewicht von nicht mehr als 2000,
Zugabe eines synthetischen Peptidsubstrates und colorimetrisches oder fluorometrisches Messen der Menge des Zersetzungsproduktes des durch die Einwirkung von PCa gebildeten Zersetzungsproduktes.

2. Verfahren zur Bestimmung von PC gemäß Anspruch 1, bei dem der PC-Aktivator ein Thrombin-Thrombomodulin-Komplex ist.

3. Verfahren zur Bestimmung von PC gemäß Anspruch 1, bei dem das Heparin in Kombination mit Antithrombin III verwendet wird.

4. Verfahren zur Bestimmung von PC gemäß Anspruch 1, bei dem der Thrombininhibitor ein N²-Arylsulfonyl-L-argininamid ist.

5. Verfahren zur Bestimmung von PC gemäß Anspruch 1, bei dem der Thrombininhibitor ein N-Arylsulfonylglycyl-amidinophenylalanin ist.

6. Ein Set zum Messen der PC-Aktivität umfassend (A) einen PC -Aktivator, (B) Antithrombin III,
(C) einen Thrombininhibitor mit einem Molekulargewicht von nicht mehr als 2000 und (D) ein synthetisches Peptidsubstrat.

7. Ein Set zum Messen der PC-Aktivität gemäß Anspruch 6, bei dem der Thrombininhibitor ein N²-Arylsulfonyl-L-argininamid ist.

8. Ein Set zum Messen der PC-Aktivität gemäß Anspruch 6, bei dem der Thrombininhibitor ein N-Arylsulfonylglycyl-amidinophenylalanin ist.

9. Ein Set zum Messen der PC-Aktivität gemäß Anspruch 6, bei dem das Set weiterhin eine Pufferlösung zur Verdünnung von Plasma und/oder einen Reaktionsbeendiger enthält.

10. Ein Set zum Messen der PC-Aktivität gemäß Anspruch 9, bei dem der PC -Aktivator ein Thrombin/Thrombomodulin-Komplex ist.

11. Ein Set zum Messen der PC-Aktivität gemäß Anspruch 10, bei dem Heparin in Kombination mit Antithrombin III verwendet wird.

## Revendications

1. Méthode enzymatique de dosage de la protéine C (appelée PC par la suite) qui comprend les étapes consistant à:
diluer au moins 10 fois un échantillon de plasma avec une solution de tampon,
ajouter un activateur de PC à l'échantillon de plasma dilué pour former la PC activée (appelée PCa par la suite)
ajouter une antithrombine III et un inhibiteur de thrombine ayant un poids moléculaire ne dépassant pas 2000,
ajouter un substrat peptidique de synthèse, et mesurer par colorimétrie ou fluorométrie la quantité de produit de décomposition formé par l'action de la PCa.

2. Méthode de dosage de la PC telle que définie dans la revendication 1, dans laquelle ledit activateur de PC est un complexe de thrombine-thrombomoduline.

3. Méthode de dosage de la PC telle que définie dans la revendication 1, dans laquelle de l'héparine est utilisée en combinaison avec l'antithrombine III.

4. Méthode de dosage de la PC telle que définie dans la revendication 1, dans laquelle ledit inhibiteur de thrombine est un N²-arylsulfonyl-L-argininamide.

5. Méthode de dosage de la PC telle que définie dans la revendication 1, dans laquelle ledit inhibiteur de thrombine est une N-arylsulfonylglycyl-amidinophénylalanine.

6. Trousse de mesure de l'activité de la PC comprenant (A) un activateur de PC, (B) une antithrombine III, (C) un inhibiteur de thrombine ayant un poids moléculaire ne dépassant pas 2000, et (D) un substrat peptidique de synthèse.

7. Trousse de mesure de l'activité de la PC telle que définie dans la revendication 6, dans laquelle ledit inhibiteur de thrombine est un N²-arylsulfonyl-L-argininamide.

8. Trousse de mesure de l'activité de la PC telle que définie dans la revendication 6, dans laquelle ledit inhibiteur de thrombine est une N-arylsulfonylglycyl-amidinophénylalanine.

9. Trousse de mesure de l'activité de la PC telle que définie dans la revendication 6, dans laquelle la trousse comprend en plus une solution de tampon pour diluer le plasma et/ou un agent pour mettre fin à la réaction.

10. Trousse de mesure de l'activité de la PC telle que définie dans la revendication 9, laquelle ledit activateur de PC est un complexe de thrombine/thrombomoduline.

11. Trousse de mesure de l'activité de la PC telle que définie dans la revendication 10, dans laquelle de l'héparine est utilisée en combinaison avec l'antithrombine III.
